(19) Europäisches Patentamt / European Patent Office / Office européen des brevets

(11) **EP 1 727 518 B1**

(12) **EUROPÄISCHE PATENTSCHRIFT**

(45) Veröffentlichungstag und Bekanntmachung des Hinweises auf die Patenterteilung:
**16.07.2008 Patentblatt 2008/29**

(21) Anmeldenummer: **05715318.1**

(22) Anmeldetag: **12.02.2005**

(51) Int Cl.:
***A61K 8/97*** *(2006.01)* ***A61K 8/49*** *(2006.01)*
***A61K 8/92*** *(2006.01)* ***A61Q 5/00*** *(2006.01)*
***A61Q 5/06*** *(2006.01)*

(86) Internationale Anmeldenummer:
**PCT/EP2005/001442**

(87) Internationale Veröffentlichungsnummer:
**WO 2005/097047 (20.10.2005 Gazette 2005/42)**

(54) **VERWENDUNG VON MITTELN ZUR KOSMETISCHEN BEHANDLUNG VON MENSCHLICHEN HAAREN**

USE OF AGENTS FOR THE COSMETIC TREATMENT OF HUMAN HAIR

UTILISATION D'AGENTS DESTINES AU TRAITEMENT COSMETIQUE DE CHEVEU HUMAIN

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **20.03.2004 DE 102004013798**

(43) Veröffentlichungstag der Anmeldung:
**06.12.2006 Patentblatt 2006/49**

(73) Patentinhaber: **Wella Aktiengesellschaft**
**64274 Darmstadt (DE)**

(72) Erfinder:

- **KRIPP, Thomas**
  **64407 Fränikisch-Crumbach (DE)**
- **GRASSER, Beate**
  **65795 Hattersheim (DE)**
- **SALLWEY, Anette**
  **63303 Dreieich (DE)**

(56) Entgegenhaltungen:
**EP-A- 0 289 639** **WO-A-00/40559**
**WO-A-90/12560** **DE-A1- 3 915 535**

- **"Das Haar und seine Struktur" 1999, WELLA AG , DARMSTADT, DE * Seite 34 - Seite 35 ***

EP 1 727 518 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gegenstand der vorliegenden Erfindung ist die Verwendung eines Mittels enthaltend eine Verbindung gemäß Formeln (I) und/oder (II)

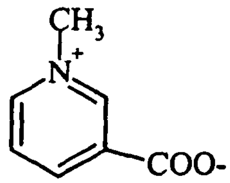

(I)

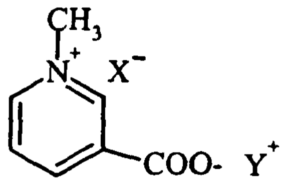

(II)

zur Reparatur, Stärkung, Restrukturierung und zum Schutz vor mechanischen und chemischen Schädigungen von menschlichen Haaren.

Die WO 00/40559 A1 offenbart Mittel zur Behandlung von Hautkrankheiten, u. a. Akne, Haarausfall und Psoriasis, gekennzeichnet durch einen Gehalt an einer Verbindung einer allgemeinen Formel (I), welche u. a. 1-Methylnikotinsäure (Trigonellin) umfasst.

Die WO 90/12560 A1 offenbart Mittel und Verfahren zur Pflege von Nägeln, Haut und Haaren, zur Bekämpfung fortschreitenden Haarausfalls und zur Anregung neuen Haarwuchses von Humanhaar, wobei eine Kombination von 80 bis 500 mg Trigonellin und 1 bis 5 mg Vitamin B6 verwendet wird.

Die Schädigung von Keratin enthaltenden Material, insbesondere keratinischen Fasern, durch Umwelteinflüsse (beispielsweise energiereiche Strahlungen), den physiologischen Status (beispielsweise Alter oder Gesundheit der betreffenden Individuen) oder mechanische und chemische Einwirkungen sind bekannt. Die Folgen sind nachteilige mechanische Eigenschaften der betroffenen Materialien. Derartige Schädigungen der inneren Struktur von keratinischen Fasern zeigen sich zum Beispiel durch Verlust an Härte, Stärke, Bruchfestigkeit, Reißfestigkeit oder Bündelzugfestigkeit.

Menschliche Haare sind üblicherweise einer Vielzahl schädigender Einflüsse ausgesetzt wie Sonnenlicht, Hitze, Chlorwasser, Meerwasser, mechanischer Belastung durch Kämmen, Bürsten, Toupieren, Frottieren, Waschen sowie gegebenenfalls durch Dauerwellbehandlungen und Anwendung kosmetischer Mittel zum Bleichen oder Färben. Während mechanische Belastungen dabei vorwiegend die Kutikula schädigen, greifen Hitze, Strahlung, alkalische, reduktive und oxidative Behandlungen dagegen direkt in die chemische Struktur des Haares ein und schädigen je nach Disposition, Art des Einflusses, Konzentration und Einwirkzeit die Haare unterschiedlich stark. Als besonders fatal zeigt sich dabei, dass Haare mit steigendem Grad der Vorschädigung auf jeden weiteren strapazierenden Einfluss um so empfindlicher reagieren. So wird beispielsweise eine Dauerwellbehandlung bei korrekter Anwendung gut vom ungeschädigten Haar vertragen; ist das Haar jedoch durch wiederholtes Bleichen stark vorgeschädigt, kann es vorkommen, dass die Dauerwellbehandlung zu einer deutlich sichtbaren Schädigung bis hin zum Abbrechen einzelner Haare führt.

Besonders an Keratinfasern, insbesondere an menschlichen Haaren, machen sich solche Einflüsse insbesondere durch verminderte Reißkraft und schlechte Kämmbarkeit bemerkbar. Hervorgerufen werden sie durch Alterungsprozesse, die vor allem physiologisch bedingt sind oder durch physikalische (Bewitterung), mechanische (Kämmen, Bürsten) und chemische (Färben, Verformen) Einflüsse induziert werden. Bei längeren Haaren machen sich diese Einflüsse insbesondere in den Haarspitzen bemerkbar. Zu den chemischen Einflüssen gehören vor allem das Bleichen, oxidatives Färben und Dauerwellen der Haare, wobei aggressive Oxidations- bzw. Reduktionsmittel zudem bevorzugt in stark alkalischem Milieu angewendet werden und dort ihre volle Wirkung zeigen. Aber auch andere chemische Einflüsse entfalten schädigende Wirkungen auf Keratin enthaltendes Material, beispielsweise mit Chlor oder Salzen angereichertes Wasser.

Es ist weder möglich noch wünschenswert, insbesondere die Haare konsequent vor den genannten Einflüssen zu schützen. Nach dem heutigen Stand der Technik ist zudem keine Methode der Haarumformung oder Farbänderung bekannt, die ihre Wirkung entfaltet ohne ein gewisses Maß an Schädigung am Ort der Behandlung zu hinterlassen. Da Haare nach ihrer Entstehung keinen biologischen Regenerationsprozessen unterworfen sind, unterliegen sie ausschließlich den äußeren Einflüssen. Es wäre daher auch wünschenswert, eine effektive Möglichkeit zu haben, die unerwünschten Begleiterscheinungen gewisser haarkosmetischer Behandlungen zu unterdrücken, jedoch unter uneingeschränkter Beibehaltung der Produktleistung.

Handelsübliche Spülungen und Kuren enthalten als Aktivsubstanzen hauptsächlich kationische Tenside bzw.

Polymere, Wachse und/oder Öle. Je geschädigter das Haar ist, desto mehr anionische Gruppen finden sich an der Oberfläche. Kationische Verbindungen werden auf dieser entgegengesetzt geladenen Oberfläche elektrostatisch angezogen, während Öle und Wachse mit den hydrophoben Gruppen des Keratins wechselwirken. Eine Strukturverbesserung im Haarinneren lässt sich mit diesen Pflegeprodukten daher nicht erreichen.

Die der vorliegenden Erfindung zu Grunde liegende Aufgabe bestand darin, ein Mittel, insbesondere ein kosmetisches Haarbehandlungsmittel, für die Verwendung zur Verbesserung des Haarzustandes bereitzustellen, das die vorgenannten Nachteile beseitigt.

Erfindungsgemäß wird diese Aufgabe gelöst durch die Verwendung einer Verbindung gemäß Formeln (I) und/oder (II)

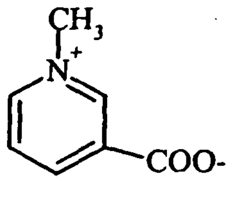

(I)

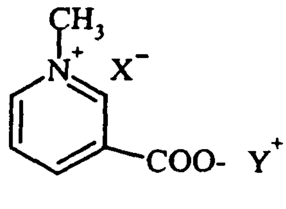

(II)

einschließlich seiner Salze, wobei $X^-$ bzw. $Y^+$ ein beliebiges anorganisches oder organisches, ein- oder mehrwertiges, physiologisch unbedenkliches Anion bzw. Kation, bedeutet, in einem Mittel zur Reparatur, Stärkung, Restrukturierung und zum Schutz vor mechanischen und chemischen Schädigungen von menschlichen Haaren.

Für $X^-$ können als Gegenion alle denkbaren organischen Säurereste (Basen) stehen. Insbesondere kann $X^-$ ausgewählt sein aus der Gruppe bestehend aus Formiat, Tartrat, Oxalat, Aspartat, Glutamat, Acetat, Citrat oder auch anorganischen Resten wie Chlorid, Bromid, Jodid, Sulfat, Hydrogensulfat, Phosphat, Mono- oder Dihydrogenphosphat, Hydroxid, Carbonat und Nitrat.

In einer bevorzugten Ausführungsform bedeutet $X^-$ Hydrochlorid, Acetat oder Citrat.

Für $Y^+$ können Kationen stehen ausgewählt aus der Gruppe bestehend aus Protonen, Alkalimetallen, vorzugsweise Lithium, Natrium, Kalium, Erdalkalimetallen, vorzugsweise Calcium und Magnesium, Nebengruppenmetallen, vorzugsweise Aluminium, Eisen, Zink, Kupfer, Mangan, Silber, Ammoniumgruppen oder primären, sekundären, tertiären oder quaternären Aminen, Hydraziden oder Hydroxylammoniumgruppen.

Weitere Ausführungsformen der vorliegenden Erfindung sind in den Unteransprüchen wiedergegeben.

Bei der Verbindungen der Formeln (I) und (II) handelt es sich um das bekannte und literaturbeschriebene Alkaloid Trigonellin (1-Methylpyridinium-3-carboxylat), die auch unter den Bezeichnungen 3-Carboxy-1-methylpyridinium-hydroxid, Nicotinsäure-N-methylbetain oder Coffearin, beschrieben ist. Insofern umfassen die in vorliegender Erfindung genannte Verbindungen (I) und (II) die Bezeichungen 1-Methylpyridinium-3-carboxylat, Trigonellin, Coffearin, 3-Carboxy-1-methylpyridinium-hydroxid, Nicotinsäure-N-methylbetain in synonymer Weise.

Die Verbindungen der Formeln (I) und (II) können entweder auf synthetischem Wege hergestellt oder nach bekannten Verfahren aus verschiedenen Pflanzenextrakten gewonnen werden, beispielsweise aus Kaffeebohnen (*Coffea arabica*) oder aus den Samen von Bockshornklee (*Trigonella foenum graecum*).

Im letzteren Fall kann die erfindungsgemäße Verwendung darin liegen, dass die Verbindungen gemäß Formeln (I) und (II) als Bestandteil eines natürlicher Pflanzenextrakts vorliegen.

Bei der Gewinnung eines Wirkstoffs einer Verbindung gemäß Formeln (I) oder (II) bzw. dem Alkaloid Trigonellin aus natürlichen Quellen bieten sich vorzugsweise ungeröstete Kaffeebohnen (*Coffea arabica*) oder Bockshornkleesamen *(Trigonella foenum-graecum*) an.

Für die erfindungsgemäße Wirkung von Verbindungen der Formeln (I) und (II) ist es unerheblich, ob sie als synthetisch gewonnene Reinsubstanz, als aufgereinigtes Isolat aus natürlichen Quellen oder als Rohextrakt eingesetzt werden. Entscheidend ist lediglich der Gehalt an Aktivsubstanz bzw. Wirkstoff. Dabei kann der Wirkstoff in seiner reinen, betainischen Form gemäß Formel (I) eingesetzt werden oder als Salz gemäß Formel (II), wie bereits beschrieben, vorliegen.

In Versuchen zur Wirkung von Verbindungen der Formeln (I) und/oder (II) hatte sich gezeigt, dass, trotz dessen betainischer Struktur, keine der klassischen Pflegewirkungen auf Haaren zu beobachten war.

Dies steht im krassen Gegensatz zu den bekannten Pflegeleistungen anderer Vertreter dieser Stoffklasse wie zum Beispiel des Betains (Trimethylammonioacetat) oder des Carnitins (3-Hydroxy-4-(trimethylammonio)-buttersäure). Während die beiden letztgenannten Verbindungen in geeigneten Formulierungen eine ausgeprägte Kämmbarkeitsverbesserung bewirken und auch den Glanz von Haaren sichtbar erhöhen, konnte mit Hilfe von Verbindungen der Formeln (I)

und (II) keine dieser Wirkungen beobachtet werden. Auch andere Pflegekriterien wie die Erhöhung des Haarvolumens, Verbesserung des Anfühlens oder der Frisurerstellung wurden nicht festgestellt.
Überraschend wurde jedoch gefunden, dass der Einsatz von Verbindungen der Formeln (I) und/oder (II) in haarkosmetischen Produkten dazu führt, dass sich die Resistenz der Haare gegenüber mechanischen und chemischen (insbesondere oxidativen und alkalischen) Angriffen erheblich verbessert.
So konnte bei Messungen des Scheuerbruchs, der ein Maß für die mechanische Strapazierfähigkeit darstellt, eine signifikante Zunahme der Lebensdauer mit Verbindungen der Formeln (I) und/oder (II) behandelten Haaren festgestellt werden (Figur 1). Bei Versuchen zur Bestimmung der triboelektrischen Eigenschaften zeigte sich außerdem, dass abhängig von der Einsatzkonzentration an Verbindungen der Formeln (I) und (II), die elektrostatische Aufladbarkeit der Haare verringert wurde. Des Weiteren wurde gefunden, dass der mechanische Abrieb künstlicher Haarfarben deutlich verringert wurde, so dass die Haltbarkeit von Haartönungen durch Verbindungen der Formeln (I) und (II) erheblich verbessert werden kann. Dies ist insbesondere deshalb überraschend, weil in keinem Vergleichsversuch die jeweils Wirkstoff gemäß Verbindungen der Formeln (I) und (II) haltige Rezeptur eine Verringerung des mechanischen Reibungskoeffizienten gezeigt hatte. Wie oben erwähnt, ließ sich ja weder die Kämmbarkeit des Haares noch die Entwirrbarkeit signifikant verbessern. Die Schutzfunktion wird folglich unabhängig von typischen Pflegekriterien ausgeübt und erstreckt sich lediglich auf die Abmilderung von Wirkungen haarschädigender Einflüsse.

Folglich war es völlig überraschend, dass durch die Verwendung von Verbindungen der Formeln (I) und/oder (II) oder deren Salzen die Struktur von von keratinischen Fasern (Haaren) derartig verändert wird, dass es vorteilhafterweise zur Reparatur, Stärkung, Restrukturierung und zum Schutz vor mechanischen und chemischen Schädigungen von Haaren verwendet werden kann.

Neben dem Schutz vor mechanischen Einflüssen zeigten Präparationen mit einem Gehalt an Verbindungen der Formeln (I) und/oder (II) auch eine ausgeprägte Schutzwirkung vor chemischen, insbesondere oxidativen Belastungen. So führte ein Gehalt von 1-Methylpyridinium-3-carboxylat in Dauerwellprodukten zu einer signifikanten Erhöhung der Reißkräfte gegenüber ansonsten gleichartig behandelten Haaren. Die Reißkraft wird als Parameter zur Bestimmung der Schädigung von Haaren angesehen; je größer der notwendige Kraftaufwand ist, um ein Haar zu zerreißen, desto besser ist die Haarqualität. In Einzelfällen konnte diese Schutzwirkung so weit gehen, dass die Reißkräfte so hoch oder gar etwas höher lagen als bei den Referenzversuchen ohne Dauerwellbehandlung. Dies deutet auf einen Reparaturmechanismus hin, der deutlich über eine normale Schutzfunktion hinausgeht.

Auch die mit einer alkalischen Behandlung zwangsläufig einhergehende Haarschädigung, die sich am augenfälligsten in einer gewissen Stumpfheit, Glanzlosigkeit zeigt, konnte durch Verwendung eines Produktes mit einem Gehalt an Verbindungen der Formeln (I) und/oder (II) deutlich verringert werden. So lagen die gemessenen Glanzwerte nach der Behandlung mit einer alkalischen Tönung bei den Produkten, die Verbindungen der Formeln (I) und/oder (II) enthielten, in allen Fällen deutlich höher als bei denjenigen Haaren, die mit dem konventionellen Produkt behandelt worden waren.
Zwar zeigt der Wirkstoff gemäß Verbindungen der Formeln (I) und/oder (II) auf ungeschädigtem oder ungestresstem Haar keines der Kriterien einer klassischen Pflegewirkung (Verbesserung der Kämmbarkeit, des Volumens, des Griffs oder des Haarglanzes). Stattdessen entfaltet es jedoch eine ausgeprägte Schutzfunktion gegenüber den oben genannten Einflüssen und kann so die negativen Auswirkungen aggressiver Behandlungsmethoden abmildern, verhindern oder teilweise sogar rückgängig machen. Besonders in Dauerwellmitteln zeigt es eine Repairwirkung. Verbindungen der Formeln (I) und/oder (II) erfüllen folglich die Wirkung eines Schutzstoffes und Restrukturanten.

Damit verbunden wird nicht nur eine Restrukturierung (Repair) von geschädigten keratinischen Fasern ermöglicht, sondern auch ein protektiver Effekt, der einer Schädigung dieser Materialien vor oder während einer Exposition mit entsprechenden Noxen entgegenwirkt und sie zu verhindern oder zu vermindern vermag.

Neben diesen von durch exogene Noxen hervorgerufenen nachteiligen Veränderungen kann die erfindungsgemäße Verwendung auch bei durch physiologische Prozesse bedingte Zustände oder Änderungen der Struktur von keratinischen Fasern vorteilhafte Wirkungen entfalten; beispielsweise bei altersbedingtem brüchigem Haar oder bei feinem Haar, welches angeboren oder altersabhängig erworben sein kann (Babyhaar, Altershaar).

Die erfindungsgemäße Verwendung erfolgt, indem menschliche Haare, mit einem den Wirkstoff gemäß Verbindung der Formel (I) und/oder (II) enthaltenden Mittel in Kontakt gebracht werden und dieses nach Applikation dort verbleibt oder nach einer geeigneten Einwirkungszeit mit einem wässrigen Mittel ab- oder ausgespült wird.

Bevorzugt ist es, wenn der Wirkstoff gemäß Formeln (I) und/oder (II) in einer Menge von 0,0005 bis 30,0 Gewichtsprozent, bevorzugt 0,001 bis 20,0 Gewichtsprozent, besonders bevorzugt 0,001 bis 10,0 Gewichtsprozent, jeweils bezogen auf die Gesamtmenge des Mittels, in dem Mittel enthalten ist.

Die einzusetzende Menge an Wirkstoff hängt sehr davon ab, welche Wirkung bei welchem Schädigungsgrad von beispielsweise Haaren eintreten soll. So kann zum Beispiel mit einer Menge von 0,001 Gewichtsprozent an Wirkstoff gemäß Formeln (I) in der betainischen Form ein Reparatur- und/oder eine Schutzwirkung im Sinne der vorliegenden Erfindung erreicht werden. Bei sehr hohen Einsatzkonzentrationen von beispielsweise 20,0 Gewichtsprozent des Wirkstoffs in Form des Hydrochlorids ist eine sehr intensive Reparatur- und/oder Protektionswirkung zu erhalten. Sie kann

insbesondere an extrem geschädigten Haarer, erreicht werden oder sie kann sich vor Anwendung einer außergewöhnlich schädigenden Behandlung, wie sie beispielsweise bei wiederholten Bleichungen zu erwarten sind, in vorteilhafter Weise entfalten.

Die vorliegende Erfindung umfasst daher auch die Verwendung zur Reparatur, Stärkung, Restrukturierung und zum Schutz vor mechanischen und chemischen Schädigungen von menschlichen Haaren, welches dadurch gekennzeichnet ist, dass ein Mittel mit einem Gehalt an einer Verbindung gemäß Formeln (I) und/oder (II)

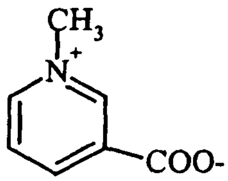

(I)

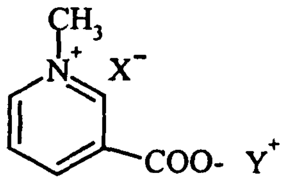

(II)

wobei $X^-$ bzw $Y^+$ ein beliebiges anorganisches oder organisches, ein- oder mehrwertiges, Anion bzw. Kation, wie oben bereits beschreiben, bedeutet, einschließlich in Form eines Pflanzenextraktes, für eine Dauer von 1 bis 60 Minuten bei einer Temperatur zwischen 10 °C und 70 °C mit dem Keratin enthaltenden Material in Kontakt gebracht und gegebenenfalls anschließend ausgespült wird.

Das für die erfindungsgemäße Verwendung beschriebene Mittel kann in allen geeigneten Formulierungen vorliegen, die in der kosmetischen oder pharmazeutischen Industrie bekannt sind.
Insbesondere kann das Mittel als wäßrige oder wässrig-alkoholische Lösung, als Gel, Creme, Emulsion oder Schaum vorliegen, wobei das Mittel sowohl in Form eines Einkomponentenpräparats als auch in Form eines Mehrkomponentenpräparates konfektioniert sein kann. Im Falle eines Einkomponentenpräparates enthält das Mittel den Wirkstoff gemäß Formeln (I) und/oder (II) zusammen mit geeigneten, dem Fachmann bekannten Hilfs- und Trägerstoffen, beispielsweise Verdicker, Säuren, Duftstoffe, Lösungsmittel, Salze, Netzmittel und/oder organischen und anorganischen UV-Filter, einzeln oder als Gemisch.

Liegt das Mittel in Form eines Mehrkomponentenpräparates vor, kann dieses aus mindestens zwei verschiedenen, bis zur Anwendung voneinander räumlich getrennten Komponenten bestehen. Eine erste Komponente kann entweder die der vorliegenden Erfindung zugrundeliegenden Verbindungen der Formeln (I) und/oder (II) als Wirkstoff für sich allein enthalten oder der Wirkstoff kann zusammen mit einem Hilfsstoff (zum Beispiel ein Verdickungsmittel) vermischt in dieser ersten Komponente vorliegen. Eine zweite oder weitere Komponente enthält nur Hilfs- und Trägerstoffe.

Es ist aber auch möglich, dass in einem Mehrkomponentenpräparat verschiedene Komponenten verschiedene Wirkstoffe gemäß der vorliegenden Erfindungen einzeln oder als Gemisch enthalten, entweder für sich allein oder zusammen mit verschiedenen Hilfsstoffen vermischt und das die weiteren Komponenten nur Hilfs- und Trägerstoffe enthalten.

Erfindungsgemäß umfasst wird die Verwendung einer Zusammensetzung, die dadurch gekennzeichnet ist, dass sie als Einkomponentenpräparat oder als Mehrkomponentenpräparat vorliegt. Liegt das verwendete Mittel als Mehrkomponentenpräparat vor, so umfasst es eine erste Komponente, die den Wirkstoff gemäß Formel (I) und/oder Formel (II) mit oder ohne Hilfs- und Zusatzstoffe enthält und eine zweite Komponente, welche die übrigen Bestandteile beinhaltet. Das verwendete Mittel kann ferner als Mehrkomponentenpräparat mit mindestens drei verschiedene Komponenten vorliegen, wobei mindestens eine der Komponenten den Wirkstoff gemäß Formeln (I) und/oder (II) enthält und die übrigen Komponenten die restlichen Bestandteile enthalten.

Es ist selbstverständlich, dass zur Herstellung eines gebrauchsfertigen Mittels die räumlich getrennt vorliegenden einzelnen Komponenten einer Mehrkomponentenpräparation kurz vor deren erfindungsgemäßen Verwendung vermischt werden müssen.
In diesem Zusammenhang sei beispielhaft auf die WO 99/11222 verwiesen, die ein entsprechendes Mehrkomponenten-System beschreibt.

Das erfindungsgemäß verwendete Mittel kann zusätzlich Träger- und Hilfsstoffe, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol; weiterhin Lösungsvermittler, Netzmittel oder Emulgatoren aus den Klassen der anionischen, kationischen, amphoteren oder nichtionogenen oberflächenaktiven Substanzen wie Fettalkoholsulfate, ethoxylierte Fettalkoholsulfate, Alkylsulfonate, Alkylbenzolsulfonate, Alkyltrimethylammoniumsalze, Alkylbetaine, ethoxylierte Fettalkohole, ethoxylierte Nonylphenole, Fettsäurealkanolamide, ethoxylierte Fettsäureester,

ferner Verdicker wie höhere Fettalkohole, Stärke oder Cellulosederivate; Salze wie z. B. NaCl; Puffersubstanzen wie Ammoniumhydrogencarbonat; Thiole, Ketocarbonsäuren (Oxocarbonsäuren), insbesondere α-Ketocarbonsäuren, bzw. deren physiologisch verträgliche Salze, UV-Absorber, Parfüme, Farbstoffe, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain; Treibmittel wie z. B. Propan, Butan, Dimethylether, $N_2O$, Luft und Kohlendioxid und Gemische davon, enthalten.

Die vorstehend erwähnten Bestandteile werden in den für solche Zwecke üblichen Mengen verwendet, zum Beispiel Wasser in einer Menge von 0,1 bis 95 Gew.%, die Netzmittel und Emulgatoren in Konzentrationen von insgesamt 0,2 bis 30 Gewichtsprozent, die Alkohole in einer Menge von insgesamt 0,1 bis 50 Gewichtsprozent, die Trübungsmittel, Parfümöle, Konservierungsstoffe und Farbstoffe in einer Menge von jeweils 0,01 bis 5 Gewichtsprozent, die Puffersubstanzen in einer Menge von insgesamt 0,1 bis 10 Gewichtsprozent, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Der pH-Wert des Mittels beträgt 2,0 bis 14,0, bevorzugt 3,0 bis 9,0. Erforderlichenfalls kann der gewünschte pH-Wert durch Zugabe von Säuren, beispielsweise α-Hydroxycarbonsäuren wie Milchsäure, Weinsäure, Zitronensäure oder Äpfelsäure, Phosphorsäure, Essigsäure, Glycolsäure, Salicylsäure oder Gluconsäurelacton, oder aber alkalisierenden Mitteln wie Ammoniak, Alkanolaminen, Alkylaminen, Alkalihydroxiden, Ammoniumhydroxiden, Alkalicarbonaten, Ammoniumcarbonaten oder Alkaliphosphaten, eingestellt werden.

Bei der Behandlung keratinischer Fasern kann das die Verbindungen gemäß Formeln (I) und/oder (II) enthaltende Mittel im Haar verbleiben oder nach der Anwendung ausgespült werden. Im letzteren Fall beträgt die Einwirkungszeit des Mittels, je nach Temperatur (etwa 20 bis 60 Grad Celsius, vorzugsweise 30 bis 50 Grad Celsius), 1 bis 60 Minuten, insbesondere 5 bis 20 Minuten, wobei durch Wärmezufuhr die Repairwirkung (Restrukturierung) beschleunigt werden kann; daher ist die Anwendung von Wärme bevorzugt. Nach Beendigung der Einwirkungszeit kann das Haar mit Wasser gespült und gegebenenfalls mit einem Shampoo gewaschen werden.

Bei den für die erfindungsgemäße Verwendung geeigneten Konfektionierungen des Mittels handelt es sich vorzugsweise um Shampoos, Spülungen, Kuren, Schäumen, Festigern, Haargelen, Haarsprays, Haarfarben, Haartönungen, Dauerwellmitteln, Fixierungen, Haarglättungsmitteln oder Brillantinen.

Das Mittel kann auch als Vorbehandlungsmittel vor chemischen und/oder physikalischen Behandlungen von Haaren, insbesondere einer Haarfärbung, einer Haartönung, einer Haarbleichung oder vor einer Haardauerverformung, verwendet werden, um einer Haarschädigung durch diese oxidativen, reduktiven, sauren oder alkalischen Behandlungen vorzubeugen.

Es konnte festgestellt werden, dass die erfindungsgemäße Verwendung eines Mittels mit einem Gehalt an der Verbindung gemäß Formel (I) und/oder (II) der vorliegenden Erfindung eine deutliche Strukturverbesserung zuvor geschädigter keratinischer Fasern ermöglicht, die sich neben den bereits beschriebenen Nachweismethoden auch mit einer statistisch hochsignifikanten Erhöhung der Reißkraft nachweisen lässt.

Die über die verschiedenen Messungen erhaltenen Resultate qualifizieren den Wirkstoff gemäß Formeln (I) und (II) eindeutig als einen Stoff, der sich in vorteilhafter Weise zur Reparatur, Stärkung, Restrukturierung und zum Schutz vor mechanischen und chemischen Schädigungen von Haaren verwenden lässt.

Legende zu den Figuren:

<u>Figur 1</u>: Messung Scheuerbruch nach getönten Haaren,

1 = Natives Haar
2 = Gebleichtes Haar
3 = Gebleichtes Haar plus Tönung
4 = Wie 3, jedoch mit 0,1 % 1-Methylpyridinium-3-carboxylat
5 = Wie 3, jedoch mit 1 % 1-Methylpyridinium-3-carboxylat

Die Messung des Scheuerbruchs erlaubt Aussagen über die Widerstandskraft des Haares gegen mechanische Belastungen. Das Messprinzip besteht darin, einzelne Haarfasem wiederholten (geringen) mechanischen Belastungen auszusetzen und die Zahl der Wiederholungs-Zyklen bis zum Ausfall des Materials zu messen. Eine höhere Zahl an Belastungszyklen zeigt eine größere Resistenz gegenüber Haarbruch durch mechanische Belastungen an. Die Auswertung der Haarbruch-Ergebnisse, die ja zunächst nur darin bestehen, dass für jedes Einzelhaar die Zahl

der Belastungszyklen bis zum Haarbruch erfasst wird, erfolgt durch die sog. Zuverlässigkeitsanalyse nach Weibull. Nach Weibull lässt sich die Ausfallhäufigkeit (hier: Haarbruch) mit folgender Gleichung beschreiben:

$$H = 1 - \exp\left(-\left(x/\Theta\right)\right)^{b}$$

linearisiert :

$$\ln\ln\left(1/\left(1-H\right)\right) = b\left(\ln x\right) - b\left(\ln\Theta\right)$$

*H*: Ausfallhäufigkeit (= kumulierte Haarbrüche), normiert auf 1 (1=100%)
*X*: Lebensdauervariable (= Zyklen bis Haarbruch)
θ: Charakteristische Lebensdauer (Zyklenzahl, bis 63,2 % der Haare gebrochen sind)
*b*: sog. Formparameter, Steigung der Ausgleichsgeraden

Für die Weibull-Analyse der Daten muss somit lnln(1/(1-*H*) gegen ln*x* aufgetragen werden. Nach linearer Regression ergibt der Achsenabschnitt *b*(lnθ), während die Steigung *b* ergibt. Die charakteristische Lebensdauer θ errechnet sich dann aus exp(-(Achsenabschnitt / Steigung)). Je größer die charakteristische Lebensdauer ist, desto größer ist die Resistenz des Haares gegen Haarbruch.

Figur 2: Messung Scheuerbruch nach Dauerwellbehandlung

1 = Natives Haar
2 = Gebleichtes Haar
3 = Gebleichtes Haar plus Dauerwelle
4 = Wie 3, jedoch mit 0,5 % 1-Methylpyridinium-3-carboxylat

Figur 3: Messung Farbabrieb

1 = Gebleichtes Haar plus Tönung
2 = Wie 1, jedoch mit 5 % 1-Methylpyridinium-3-carboxylat

Die nachfolgenden Beispiele sollen den Gegenstand näher erläutern. Die darin angegebenen Mengenangaben an Wirk-, Hilfs- und Trägerstoffen beziehen sich auf Gewichtsprozent bezogen auf das Endprodukt, wenn nicht anders angegeben.

**Beispiel 1:** Messung des Scheuerbruchs an Haaren

Als weiterer wichtiger Parameter zur Determinierung der Beschaffenheit des Zustandes von Haaren bzw. des Schädigungsgrades von Haaren ist die Messung des Scheuerbruchs als Maß für die mechanische Strapazierbarkeit eines Haares (siehe Legende zu Fig.1).

Als weiterer wichtiger Parameter zur Determinierung der Beschaffenheit des Zustandes von Haaren bzw. des Schädigungsgrades von Haaren ist die Messung des Scheuerbruchs als Maß für die mechanische Strapazierbarkeit eines Haares (siehe Legende zu Fig. 1).

Hierzu wurden menschliche Haarsträhnen (kaukasischer Herkunft), nach vorheriger standardisierter Bleichung, den stark strapazierenden Bedingungen einer Dauerwellprozedur bzw. einer Haartönung nach standardisierten Methoden ausgesetzt. Anschließend wurden je 20 Einzelhaare pro Muster, d. h. 10 Einzelhaare pro Strähne, an ihren Enden in Hülsen gefasst und der Scheuerbruch gemessen.

Die reine Haartönung (ohne 1-Methylpyridinium-3-carboxylat) bewirkt eine allenfalls minimal höhere charakteristische Lebensdauer des Haares als die des gebleichten, ansonsten unbehandelten Haares. Der Zusatz von 0,1 % 1-Methylpyridinium-3-carboxylat führt zu einer Erhöhung und der Zusatz von 1 % 1-Methylpyridinium-3-carboxylat zu einer weiteren Erhöhung der charakteristischen Lebensdauer des Haares. Die Werte des mit der 1%-igen 1-Methylpyridinium-3-carboxylat Konzentration in Haartönung behandelten Haare sind sogar größer als die des chemisch unbehandelten (ungebleichten, nativen) Haares (Fig. 1).

Dauerwellprozesse reduzieren die charakteristische Lebensdauer des Haares und somit die Widerstandskraft

gegen mechanische Belastungen. Der Zusatz von 0,5 % 1-Methylpyridinium-3-carboxylat in Dauerwellmitteln führte zu einer Erhöhung der charakteristischen Lebensdauer des Haares. Der erhaltene Wert ist sogar höher als der des gebleichten, ansonsten jedoch unbehandelten Haares (Fig. 2).

Tabelle 1: Messergebnisse zum Scheuerbruch nach Haartönung

| Muster zum Test in Haartönung (Fig. 1) | Charakteristische Lebensdauer |
|---|---|
| Nativ | 596 |
| Gebleicht | 524 |
| Haartönung pur | 530 |
| Haartönung mit + 0,1% 1-Methylpyridinium-3-carboxylat | 568 |
| Haartönung mit + 1% 1-Methylpyridinium-3-carboxylat | 643 |

Tabelle 2: Messergebnisse zum Scheuerbruch nach Dauerwellbehandlung

| Muster zum Test in Dauerwellmittel (Fig. 2) | Charakteristische Lebensdauer |
|---|---|
| Nativ | 596 |
| Gebleicht | 524 |
| Standardrezeptur Dauerwelle | 439 |
| Dauerwellrezeptur mit 0,5 % 1-Methylpyridinium-3-carboxylat | 546 |

**Beispiel 2:** Bestimmung der triboelektrischen Eigenschaften an Haaren

Auch die Bestimmung der triboelektrischen Eigenschaften, also die Messung der elektrostatischen Aufladbarkeit der Haare, lässt sich als Maß einer Reparatur- oder Schutzwirkung im Sinne der vorliegenden Erfindung heranziehen. Hierbei macht man sich die Eigenschaft zunutze, dass sich bei tribologischen Prozessen durch Elektronenwanderung Aufladungen ergeben, die gemäß einer Spannungsreihe eine positive oder negative Ladung bzw. Kontaktspannung am betreffenden Körper ergeben.

Bei der Antistatikmessung wird die elektrostatische Aufladung des mit einem Kunststoffkamm erzeugten Volumens einer Haarsträhne ermittelt, welches bildanalytisch bestimmt wird.

Verwendet wurden je 3 Volumensträhnen pro Muster, Eurohaar, Länge: 22 cm, Breite: 2.5 cm, Gewicht: 3.0 g Um eine mittlere Haarqualität zu gewährleisten, wurden die Zählhaar- und Volumensträhnen zunächst mit Bleichpulver (2,5 g / 1g Haar) und 9 %-igem Welloxon® (7,5 ml / 1g Haar) 30 Min. gebleicht, anschließend 2 Min. unter fließendem Wasser (35 °C) ausgespült, dann zweimal mit je 0,5 ml Standardshampoo pro 2 g Haare 1 Min. gewaschen, 1 Min. gespült und mindestens über Nacht im Klimaraum bei 20 °C und 65 % relative Feuchte getrocknet. Die so vorbehandelten Strähnen wurden nun mit 1-Methylpyridinium-3-carboxylat-haltiger (0,5 %) Haarkur behandelt. Nach fünfminütigem Einwirken, Ausspülen und Trocknen der Haare wurden die Haarsträhnen nun vor der Messung zehnmal mit der groben Seite eines handelsüblichen Kunststoffkammes aufgekämmt. Als Vergleich dienten 3 unbehandelte Strähnen.

Die so behandelten Haarsträhnen zeigten nach dem Aufkämmen mit einem Kunststoffkamm ein sichtbar geringeres Volumen als die unbehandelten Vergleichshaare, das bedeutet, dass die Kuren die elektrostatische Aufladung der Haare und damit auch das Haarvolumen reduzierten. Ein statistisch wahrscheinlicher Antistatik-Effekt konnte für die Rezeptur mit 1% 1-Methylpyridinium-3-carboxylat als Hydrochlorid nachgewiesen werden.

**Beispiel 3:** Bestimmung des mechanischen Abrieb künstlicher Farbstoffe am Haar

Unter Farbabrieb ist die Anfärbung von Textilien durch den Kontakt mit feuchten oder trockenen, gefärbten Haaren zu verstehen. In der Praxis wird diese Eigenschaft in der Art erlebt, dass Teile der Kleidung beim Überstreifen durch gefärbte Haare angefärbt werden können. Die Messung des Farbabriebs dient nun dazu, diesen Effekt zu bestimmen.

Das Abreiben von Haarfarben ist ein rein physikalischer Vorgang. Erfolgt der Abrieb auf trockenem Haar, so basiert die Anfärbung der Stoffe prinzipiell durch mechanisches Ablösen von Farbpartikeln vom Haar und deren Übertrag auf das Gewebe. Findet der Abrieb auf feuchtem Haar statt, wird der Effekt des mechanischen Übertrags noch vom Ausbluten der Haarsträhnen und der dadurch bedingten Anfärbung der Textilien überlagert.

Zur Messung des Farbabriebs gefärbter Haarsträhnen wurde ein Baumwollband auf einer feuchten Haarsträhne mit konstantem Druck über die Haarsträhne gerieben. Die Quantifizierung des Farbabriebs am Haar erfolgte über die bekannte Lab-Farbmessung (Reflexionsmessung mittels Minolta Spectrophotometer CM-508i). Der daraus ermittelte Farbabstand ΔE (zum weißen Textilband) ist ein Maß für die Abgabe des Farbstoffs am Haar. Je größer der ΔE - Wert, desto höher ist der Farbabrieb der Haarsträhne.

Hierzu wurde auf jeweils 3 trockene Kämmsträhnen mindestens 30 g einer handelsüblichen Haartönungsformulierung (Wella, Color Fresh Liquid) aufgetragen. Nach einer Einwirkzeit von 30 Min. bei Raumtemperatur spülte man die Haare 2 Min. unter fließendem Wasser (35 °C) aus und stellte sie auf eine Restfeuchte von 50 % ein.

Anschließend wurde bei konstantem Druck ein weißes Baumwollband in 300 Zyklen über jede der gefärbten Strähnen gerieben. Zur Ermittlung des Farbabriebs führte man am nächsten Tag die Lab-Farbmessung durch (Fig. 3).

Tabelle 3: Messwerte des Farbabriebs

| | Gehalt an 1-Methylpyridinium-3-carboxylat als Hydrochlorid | Delta E-Wert (Fig. 3) |
|---|---|---|
| Probe 1 | - | 28,97 |
| Probe 2 | 5 % | 26,83 |

**Beispiel 4:** Ermittlung der Reißkraft an Haaren

Die Ermittlung der Reißkraft von Haaren, die ein Indikator für die strukturelle Integrität des Haarcortex und damit ein Maß für den Schädigungsgrad ist, erfolgt durch für diese Zwecke übliche Zug-Dehnungs-Messungen. Von jeder Haarsträhne werden 20 Einzelhaare ausgewählt und die einzelnen Haardurchmesser mit einem computergesteuerten Lasermikrometer bestimmt. Anschließend wird mit einem Zug-Dehnungs-Meßgerät (MTT 160/600 Series Miniature Tensile Tester, Serial No. 600.95.05.001, Fa. DIA-STRON Ltd., England) die Kraft gemessen, die nötig ist, um die einzelnen Haare zum Zerreißen zu bringen.

Aus diesen einzelnen, aufgrund der unterschiedlichen Haardurchmesser voneinander abweichenden Reißkraft-Meßwerten wird die sog. Bündelzugfestigkeit (BZF) ermittelt, indem aus den Einzelwerten unter Berücksichtigung der jeweiligen Haardurchmesser die Reißkraft für einen Haardurchmesser von 0,08 mm (mittlerer Durchmesser) errechnet wird. Durch Einbeziehung der Haardichte erfolgt schließlich die Umrechnung in die Einheit der Bündelzugfestigkeit (cN/tex). Je größer der Zahlenwert der Bündelzugfestigkeit, desto geringer ist die Haarschädigung.

Die Messungen an Haaren, die mit einem 1-Methylpyridinium-3-carboxylat haltigen und einem herkömmlichen Shampoo ohne 1-Methylpyridinium-3-carboxylat behandelt wurden, ergaben folgende Ergebnisse:

Geschädigtes Haar (blondiert) wurde behandelt mit dem Shampoo nach Beispiel 2 jedoch ohne 1-Methylpyridinium-3-carboxylat als Hydrochlorid. Die Menge an 1-Methylpyridinium-3-carboxylat wurde durch Wasser ersetzt. In diesem Fall betrug die BZF = 12,0 +/- 0,4 cN/tex (ermittelt an 17 Haaren aus einer Shampoobehandelten Strähne);

Geschädigtes Haar (blondiert) behandelt mit dem Shampoo gemäß nachfolgendem Beispiel 6 und mit 2,0 Gew.% 1-Methylpyridinium-3-carboxylat zeigte eine BZF = 12,64 +/- 0,4 cN/tex (ermittelt an 20 Haaren aus einer Shampoobehandelten Strähne).

Der Unterschied zwischen den oben angegebenen Mittelwerten ist statistisch hochsignifikant (Signifikanzniveau ermittelt mit t-Test: 99,9%).
Die Bündelzugfestigkeit wird durch den Gehalt an 2,0 Gew.% 1-Methylpyridinium-3-carboxylat von 12,0 cN/tex auf 12,64 cN/tex erhöht. Der Zusatz von 1-Methylpyridinium-3-carboxylat bedingt somit eine deutliche Haarstärkung bzw. Repairwirkung.

**Beispiele 5 - 7:** Haarschutzshampoos

| | Mengen in Gew.% | | |
|---|---|---|---|
| | **5** | **6** | **7** |
| Natriumlaurylethersulfat (25%) | 40,0 | 40,0 | 40,0 |
| NaCl | 4,0 | 4,0 | 4,0 |

(fortgesetzt)

| | Mengen in Gew.% | | |
|---|---|---|---|
| | **5** | **6** | **7** |
| 1-Methylpyridinium-3-carboxylat hydrochlorid | 0,2 | 2,0 | 5,0 |
| Wasser | ad 100 | ad 100 | ad 100 |

**Beispiel 8 :** Haarshampoo für intensiven Schutz

| | Mengen in Gew.% |
|---|---|
| Natriumlaurylethersulfat (25%) | 35,0 |
| NaCl | 2,0 |
| Triethanolamin | 4,0 |
| Phenoxyethanol | 0,08 |
| Methyldibromoglutaronitrile | 0,02 |
| Parfümöl | 0,1 |
| 1-Methylpyridinium-3-carboxylat hydrochlorid | 20,0 |
| Wasser | ad 100 |

**Beispiele 9 -11:** Haarschutzsprays

| | Mengen in Gew.% | | |
|---|---|---|---|
| | **9** | **10** | **11** |
| Vinylacetat/Crotonsäure-Copolymer | 2,0 | 2,0 | 2,0 |
| 2-Amino-2-methyl-1-propanol | 0,160 | 0,160 | 0,160 |
| Ethanol (96%) | 37,840 | 37,840 | 37,840 |
| 1-Methylpyridinium-3-carboxylat (betainische Form) | 0,001 | 0,005 | 0,010 |
| Parfümöl | 0,100 | 0,100 | 0,100 |
| Propan / Butan (60 : 40) | ad 100 | ad 100 | ad 100 |

**Beispiele 12 - 16:** Cremeshampoos für dauergewelltes Haar

| | Mengen in Gew.% | | | | |
|---|---|---|---|---|---|
| | **12** | **13** | **14** | **15** | **16** |
| Natriumlaurylsulfat | 10,0 | | | | |
| Natriummyristylsulfat | | 10,0 | | | |
| Natriumcetylsulfat | | | 10,0 | | |
| Natriumstearylsulfat | | | | 10,0 | |
| Laureth-10 | | | | | 10,0 |

(fortgesetzt)

| | Mengen in Gew.% | | | | |
|---|---|---|---|---|---|
| | **12** | **13** | **14** | **15** | **16** |
| Stearinsäure | 9,0 | 9,0 | 9,0 | 9,0 | 9,0 |
| 1-Methylpyridinium-3-carboxylat als Acetat | 1,0 | 1,0 | 1,0 | 1,0 | 1,0 |
| NaCl | 3,0 | 3,0 | 3,0 | 3,0 | 3,0 |
| Triäthanolamin, rein | 4,0 | 4,0 | 4,0 | 4,0 | 4,0 |
| 1,2-Dibrom-2,4-dicyanobutanin-2-phenoxyethanol | 0,1 | 0,1 | 0,1 | 0,1 | 0,1 |
| Wasser | ad 100 | ad 100 | ad 100 | ad100 | ad100 |

**Beispiele 17 - 21:** Cremeshampoos für dauergewelltes Haar

| | Mengen in Gew.% | | | | |
|---|---|---|---|---|---|
| | **17** | **18** | **19** | **20** | **21** |
| Natriumlaurylsulfat | 10,0 | | | | |
| Natriummyristylsulfat | | 10,0 | | | |
| Natriumcetylsulfat | | | 10,0 | | |
| Natriumstearylsulfat | | | | 10,0 | |
| Laureth-10 | | | | | 10,0 |
| Stearinsäure | 9,0 | 9,0 | 9,0 | 9,0 | 9,0 |
| 1-Methylpyridinium-3-carboxylat als Acetat | 3,0 | 3,0 | 3,0 | 3,0 | 3,0 |
| NaCl | 3,0 | 3,0 | 3,0 | 3,0 | 3,0 |
| Triäthanolamin, rein | 4,0 | 4,0 | 4,0 | 4,0 | 4,0 |
| 1,2-Dibrom-2,4-dicyanobutanin-2-phenoxyethanol | 0,1 | 0,1 | 0,1 | 0,1 | 0,1 |
| Wasser | ad 100 | ad 100 | ad 100 | ad100 | ad100 |

**Beispiele 22 - 26:** Cremeshampoos für dauergewelltes Haar

| | Mengen in Gew.% | | | | |
|---|---|---|---|---|---|
| | **22** | **33** | **24** | **25** | **26** |
| Natriumlaurylsulfat | 10,0 | | | | |
| Natriummyristylsulfat | | 10,0 | | | |
| Natriumcetylsulfat | | | 10,0 | | |
| Natriumstearylsulfat | | | | 10,0 | |
| Laureth-10 | | | | | 10,0 |
| Stearinsäure | 9,0 | 9,0 | 9,0 | 9,0 | 9,0 |
| 1-Methylpyridinium-3-carboxylat als Acetat | 6,0 | 6,0 | 6,0 | 6,0 | 6,0 |
| NaCl | 3,0 | 3,0 | 3,0 | 3,0 | 3,0 |

(fortgesetzt)

| | Mengen in Gew.% | | | | |
|---|---|---|---|---|---|
| | **22** | **33** | **24** | **25** | **26** |
| Triäthanolamin, rein | 4,0 | 4,0 | 4,0 | 4,0 | 4,0 |
| 1,2-Dibrom-2,4-dicyanobutanin-2-phenoxyethanol | 0,1 | 0,1 | 0,1 | 0,1 | 0,1 |
| Wasser | Ad 100 | ad 100 | ad 100 | ad100 | ad100 |

**Beispiele 27 - 31:** Cremeshampoos für dauergewelltes Haar

| | Mengen in Gew.% | | | | |
|---|---|---|---|---|---|
| | **27** | **28** | **29** | **30** | **31** |
| Natriumlaurylsulfat | 10,0 | | | | |
| Natriummyristylsulfat | | 10,0 | | | |
| Natriumcetylsulfat | | | 10,0 | | |
| Natriumstearylsulfat | | | | 10,0 | |
| Laureth-10 | | | | | 10,0 |
| Stearinsäure | 9,0 | 9,0 | 9,0 | 9,0 | 9,0 |
| 1 -Methylpyridinium-3-carboxylat als Acetat | 10,0 | 10,0 | 10,0 | 10,0 | 10,0 |
| NaCl | 3,0 | 3,0 | 3,0 | 3,0 | 3,0 |
| Triäthanolamin, rein | 4,0 | 4,0 | 4,0 | 4,0 | 4,0 |
| 1,2-Dibrom-2,4-dicyanobutanin-2-phenoxyethanol | 0,1 | 0,1 | 0,1 | 0,1 | 0,1 |
| Wasser | ad 100 | ad 100 | ad 100 | ad100 | Ad100 |

**Beispiele 32 - 34:** Restrukturierungs-Intensivhaarkur

| | Mengen in Gew.% | | |
|---|---|---|---|
| | **32** | **33** | **34** |
| Glycerinmonostearat, neutral | 6,0 | 6,0 | 6,0 |
| Lanolinalkoxylat | 2,0 | 2,0 | 2,0 |
| 1-Methylpyridinium-3-carboxylat als Citrat | 1,0 | 5,0 | 9,0 |
| Cetylalkohol | 2,0 | 2,0 | 2,0 |
| Gemisch aus Lanolinalkohol und Paraffinöl (1 : 9) | 1,0 | 1,0 | 1,0 |
| Quaternium-52 | 1,5 | 1,5 | 1,5 |
| Hydroxyethylcellulose | 0,2 | 0,2 | 0,2 |
| Citronensäure | 0,1 | 0,1 | 0,1 |
| Sorbinsäure | 0,2 | 0,2 | 0,2 |
| Wasser | ad 100 | ad 100 | ad 100 |

**Beispiele 35 - 36:** Haarkur für blondiertes Haar

| | Mengen in Gew.% | |
|---|---|---|
| | **35** | **36** |
| Glycerinmonostearat | 6,0 | 6,0 |
| Lanolinalkoxylat | 2,0 | 2,0 |
| Cetylalkohol | 2,0 | 2,0 |
| Gemisch aus Lanolinalkohol und Paraffinöl (1 : 9) | 1,0 | 1,0 |
| Tris-(oligooxyethyl)-alkyl-ammoniumphosphat | 1,5 | 1,5 |
| Hydroxyethylcellulose | 0,2 | 0,2 |
| Citronensäure | 0,1 | 0,1 |
| Sorbinsäure | 0,1 | 0,1 |
| Parfümöl | 0,1 | 0,1 |
| 1-Methylpyridinium-3-carboxylat hydrochlorid | 0,5 | 1,5 |
| Wasser | ad 100 | ad 100 |

**Beispiel 37 :** Dauerwellfixierungslösung mit Repairfunktion

| | Mengen in Gew.% |
|---|---|
| Wasserstoffperoxid | 4,6 |
| Citronensäure | 0,2 |
| 1-Methylpyridinium-3-carboxylat hydrochlorid | 0,5 |
| Parfümöl | 0,1 |
| Wasser | ad 100 |

**Beispiele 38 - 39:** Schaumkonditioner zum Schutz vor Kämm-Schäden

| | Mengen in Gew.% | |
|---|---|---|
| | **38** | **39** |
| Polyquaternium-16 | 5,00 | 5,00 |
| VP/VA-Copolymer | 1,00 | 1,00 |
| Ceteareth-12 | 0,15 | 0,15 |
| Parfümöl | 0,10 | 0,10 |
| 1-Methylpyridinium-3-carboxylat hydrochlorid | 1,00 | 2,00 |
| Propan/Butan (60 : 40) | 10,00 | 10,00 |
| Wasser | ad 100 | ad 100 |

**Beispiele 40 - 41:** Dauerwellrezeptur mit geringer Haarschädigung

| | Mengen in Gew.% | |
|---|---|---|
| | **40** | **41** |
| Ammoniumthioglykolat (80%) | 9,5 | 9,5 |
| Ammoniak (25%) | 1,6 | 1,6 |
| Ammoniumcarbonat | 4,5 | 4,5 |
| 1-Methylpyridinium-3-carboxylat (Betainform) | 0,5 | 3,0 |
| Parfümöl | 0,2 | 0,2 |
| Wasser | ad 100 | ad 100 |

**Beispiele 42 - 43:** O/W Frisiercreme mit Repairfunktion

| | Mengen in Gew.% | |
|---|---|---|
| | **42** | **43** |
| Alkyletherphosphat | 3,0 | 3,0 |
| Polyquaternium-22 | 1,2 | 1,2 |
| Paraffinöl | 17,0 | 17,0 |
| Parfümöl | 0,3 | 0,3 |
| Triethanolamin | 1,5 | 1,5 |
| Natriumformiat | 0,5 | 0,5 |
| Extrakt aus *Trigonella foenum-graecum*[1] | 0,2 | 1,0 |
| Wasser | ad 100 | ad 100 |
| [1] Wässriger Extrakt, deproteiniert, entfettet und aufkonzentriert. Aktivgehalt, berechnet als 1-Methylpyridinium-3-carboxylat in betainischer Form, 10 %. | | |

Ein Kilogramm gemahlener Bockshornkleesamen wurde mit fünf Litern Äthanol / Wasser (60 % Äthanol) zwei Stunden unter Rückfluss erhitzt. Anschließend wurde abfiltriert und der Filterrückstand ein zweites Mal mit drei Litern 60 % Äthanol extrahiert. Aus den vereinigten Filtraten wurde der Äthanolanteil abdestilliert. Die verbleibende wässrige Phase wurde mit Ameisensäure auf pH 2 bis 3 angesäuert und zur Entfernung der Fette dreimal mit je einem Liter Essigsäureäthylester ausgeschüttelt. Die wässrige Phase wurde nun auf ca. 10 g eingeengt und der exakte Gehalt an 1-Methylpyridinium-3-carboxylat mittels HPLC bestimmt. Anschließend wurde durch weiteres Aufkonzentrieren oder durch Wasserzugabe der Wirkstoffgehalt auf 10 % eingestellt. Man erhielt auf diese Weise zwischen 8 und 15 g Extrakt. Die verbliebene Rest-Ameisensäure wirkte gleichzeitig konservierend.

**Beispiele 44 - 45:** Haarfestiger für langes Haar

| | Mengen in Gew.% | |
|---|---|---|
| | **44** | **45** |
| Luviskol VA 55E | 6,0 | 6,0 |

(fortgesetzt)

| | Mengen in Gew.% | |
|---|---|---|
| | **44** | **45** |
| Parfümöl | 0,3 | 0,3 |
| Ethanol (96%) | 40,0 | 40,0 |
| 1-Methylpyridinium-3-carboxylat hydrochlorid | 0,2 | 2,0 |
| Wasser | ad 100 | ad 100 |

**Beispiele 46 - 47:** Tönungsschaum mit geringem Farbabrieb

| | Mengen in Gew.% | |
|---|---|---|
| | **46** | **47** |
| Ethanol (96%) | 10,0 | 10,0 |
| Polyquaternium-11 | 7,5 | 7,5 |
| Basic Violet 2 | 0,2 | 0,2 |
| Cyclomethicone | 0,2 | 0,2 |
| Parfümöl | 0,3 | 0,3 |
| 1-Methylpyridinium-3-carboxylat hydrochlorid | 0,5 | 2,5 |
| Wasser | ad 100 | ad 100 |
| Propan / Butan (60 : 40) | 10,0 | 10,0 |

**Beispiele 48 - 49:** Cremehaarfarbe für geringen Farbabrieb

| | Mengen in Gew.% | |
|---|---|---|
| | **48** | **49** |
| Stearylalkohol | 8,00 | 8,00 |
| Paraffinöl | 13,00 | 13,0 |
| Wollfett | 6,00 | 6,00 |
| Parfüm | 0,30 | 0,30 |
| p-Toluendiamin | 0,70 | 0,70 |
| Resorcin | 0,05 | 0,05 |
| Aminophenol | 0,06 | 0,06 |
| EDTA | 0,20 | 0,20 |
| Ammoniak (25%) | 2,00 | 2,00 |
| Natriumsulfit | 1,00 | 1,00 |
| 1-Methylpyridinium-3-carboxylat hydrochlorid | 1,00 | 5,00 |
| Wasser | ad 100 | ad 100 |

**Beispiele 50 - 51:** Haarwasser für häufiges Kämmen

| | Mengen in Gew.% | |
|---|---|---|
| | **50** | **51** |
| Ethanol (96%) | 60,00 | 60,00 |
| Parfümöl | 0,15 | 0,15 |
| Panthenol | 0,20 | 0,20 |
| Luviskol K 30 | 0,05 | 0,05 |
| Salicylsäure | 0,10 | 0,10 |
| Menthol | 0,02 | 0,02 |
| Campher | 0,01 | 0,01 |
| Allantoin | 0,10 | 0,10 |
| Extrakt aus *Coffea arabica*[1] | 10,00 | 20,00 |
| Wasser | ad 100 | ad 100 |
| [1] Wässriger Extrakt aus gemahlenem ungerösteten Kaffeebohnen, deproteiniert, entfettet und aufkonzentriert. Aktivgehalt, berechnet als 1-Methylpyridinium-3-carboxylat in der Betainform, 10%. | | |

Ein Kilogramm gemahlener, ungerösteter Kaffeebohnen wurde mit fünf Litern Äthanol / Wasser (60 % Äthanol) zwei Stunden· unter Rückfluss erhitzt. Anschließend wurde abfiltriert und der Filterrückstand ein zweites Mal mit drei Litern 60 % Äthanol extrahiert. Aus den vereinigten Filtraten wurde der Äthanolanteil abdestilliert. Die verbleibende wässrige Phase wurde mit Ameisensäure auf pH 2 bis 3 angesäuert und zur Entfernung der Fette dreimal mit je einem Liter Essigsäureäthylester ausgeschüttelt. Die wässrige Phase wurde nun auf ca. 100 g eingeengt und der exakte Gehalt an 1-Methylpyridinium-3-carboxylat mittels HPLC bestimmt. Anschließend wurde durch weiteres Aufkonzentrieren oder durch Wasserzugabe der Wirkstoffgehalt auf 10 % eingestellt. Man erhielt auf diese Weise zwischen 60 und 150 g Extrakt. Die verbliebene Rest-Ameisensäure wirkte gleichzeitig konservierend.

## Patentansprüche

1. Verwendung einer Verbindung gemäß Formeln (I) und/oder (II)

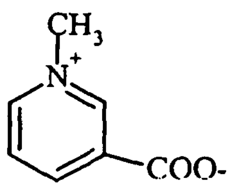

(I)

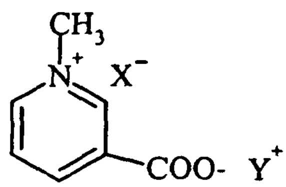

(II)

einschließlich seiner Salze, wobei $X^-$ bzw. $Y^+$ ein beliebiges anorganisches oder organisches, ein- oder mehrwertiges, physiologisch unbedenkliches Anion bzw. Kation, bedeutet, in einem Mittel zur Reparatur, Stärkung, Restrukturierung und zum Schutz vor mechanischen und chemischen Schädigungen von menschlichen Haaren.

2. Verwendung nach Anspruch 1, **dadurch gekennzeichnet, dass** die Verbindungen gemäß Formeln (I) und/oder (II) synthetischen oder natürlichen Ursprungs ist.

3. Verwendung nach Anspruch 1 bis 2, **dadurch gekennzeichnet, dass** die Verbindungen gemäß Formeln (I) und/oder (II) als Bestandteil eines natürlicher Pflanzenextrakts vorliegen.

4. Verwendung nach Ansprüchen 1 bis 3 **dadurch gekennzeichnet, dass** der Wirkstoff in seiner reinen, betainischen Form oder als Salz als Verbindung gemäß Formel (II) vorliegt.

5. Verwendung nach Ansprüchen 1 bis 4, **dadurch gekennzeichnet, dass** $X^-$ ausgewählt ist aus der Gruppe bestehend aus Formiat, Tartrat, Oxalat, Aspartat, Glutamat, Acetat, Citrat oder auch anorganischen Resten wie Chlorid, Bromid, Jodid, Sulfat, Hydrogensulfat, Phosphat, Mono- oder Dihydrogenphosphat, Hydroxid, Carbonat, Nitrat und dass $Y^-$ ausgewählt ist aus der Gruppe bestehend aus Protonen, Alkalimetallen, vorzugsweise Lithium, Natrium, Kalium, Erdalkalimetallen, vorzugsweise Calcium und Magnesium, Nebengruppenmetallen, vorzugsweise Aluminium, Eisen, Zink, Kupfer, Mangan, Silber, Ammoniumgruppen oder primären, sekundären, tertiären oder quaternären Aminen, Hydraziden oder Hydroxylammoniumgruppen.

6. Verwendung nach einem der Ansprüche 1 bis 5 bei strapaziertem und/oder geschädigtem Haar.

7. Verwendung nach einem der Ansprüche 1 bis 6, **dadurch gekennzeichnet, dass** das Mittel vor, während oder nach einer Exposition des Haares mit chemischen und/oder physikalischen Noxen mit dem Haar in Kontakt gebracht wird.

8. Verwendung nach Ansprüchen 1 bis 7 in einem Vorbehandlungsmittel vor einer chemischen und/oder physikalischen Behandlung Haar.

9. Verwendung nach einem der Ansprüche 1 bis 8 zur Verhütung oder Verminderung von Schädigungen der inneren Struktur oder zur Reparatur (Restrukturierung) von Haar.

10. Verwendung nach einem der vorhergehenden Ansprüche 1 bis 9, **dadurch gekennzeichnet, dass** die chemische Behandlung eine Färbung, Tönung, Bleichung oder eine Dauerverformung umfasst.

11. Verwendung nach einem der Ansprüche 1 bis 10 zur kosmetischen Behandlung von empfindlichen, brüchigen und/oder feinen Haaren.

12. Verwendung nach einem der Ansprüche 1 bis 11, **dadurch gekennzeichnet, dass** die Verbindungen gemäß Formeln (I) und/oder (II) in einer Menge von 0,001 bis 30,0 Gewichtsprozent, bezogen auf die Gesamtmenge, in dem Mittel enthalten sind.

13. Verwendung nach einem der Ansprüche 1 bis 12, **dadurch gekennzeichnet, dass** die Verbindungen gemäß Formeln (I) und (II) in einer Menge von 0,05 bis 10,0 Gewichtsprozent, bezogen auf die Gesamtmenge, in dem Mittel enthalten sind.

14. Verwendung nach einem der Ansprüche 1 bis 13, **dadurch gekennzeichnet, dass** das Mittel als wässrige oder wässrig-alkoholische Lösung, Emulsion, Schaum, Creme oder Gel vorliegt.

## Claims

1. Use of a compound according to formulae (I) and/or (II)

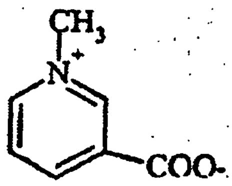

(I)

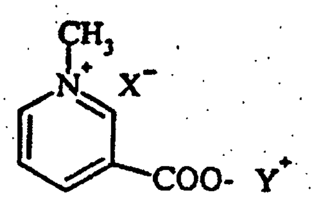

(II)

including its salts, where $X^-$ or $Y^+$ is any desired inorganic or organic, monovalent or polyvalent physiologically acceptable anion or cation, respectively, in an agent for the repair, strengthening, restructuring and protection against mechanical and chemical damage of human hair.

**2.** Use according to Claim 1, **characterized in that** the compounds according to formulae (I) and/or (II) are of synthetic or natural origin.

**3.** Use according to Claims 1 to 2, **characterized in that** the compounds according to formulae (I) and/or (II) are present as constituent of a natural plant extract.

**4.** Use according to Claims 1 to 3, **characterized in that** the active ingredient is present in its pure, betainic form or as salt as compound according to formula (II).

**5.** Use according to Claims 1 to 4, **characterized in that** $X^-$ is selected from the group consisting of formate, tartrate, oxalate, aspartate, glutamate, acetate, citrate or else inorganic radicals such as chloride, bromide, iodide, sulphate, hydrogen sulphate, phosphate, mono- or dihydrogen phosphate, hydroxide, carbonate, nitrate, and **in that** $Y^+$ is selected from the group consisting of protons, alkali metals, preferably lithium, sodium, potassium, alkaline earth metals, preferably calcium and magnesium, subgroup metals, preferably aluminium, iron, zinc, copper, manganese, silver, ammonium groups or primary, secondary, tertiary or quaternary amines, hydrazides or hydroxylammonium groups.

**6.** Use according to one of Claims 1 to 5 with stressed and/or damaged hair.

**7.** Use according to one of Claims 1 to 6, **characterized in that** the agent is brought into contact with the hair before, during or after exposing the hair to chemical and/or physical noxae.

**8.** Use according to Claims 1 to 7 in a pretreatment agent before a chemical and/or physical treatment of hair.

**9.** Use according to one of Claims 1 to 8 for the prevention or reduction of damage to the inner structure or for the repair (restructuring) of hair.

**10.** Use according to one of the preceding Claims 1 to 9, **characterized in that** the chemical treatment involves a colouring, tinting, bleaching or permanent shaping.

**11.** Use according to one of Claims 1 to 10 for the cosmetic treatment of sensitive, brittle and/or fine hair.

**12.** Use according to one of Claims 1 to 11, **characterized in that** the compounds according to formulae (I) and/or (II) are present in the agent in an amount of from 0.001 to 30.0 per cent by weight, based on the total amount.

**13.** Use according to one of Claims 1 to 12, **characterized in that** the compounds according to formulae (I) and (II) are present in the agent in an amount of from 0.05 to 10.0 per cent by weight, based on the total amount.

**14.** Use according to one of Claims 1 to 13, **characterized in that** the agent is in the form of an aqueous or aqueous-alcoholic solution, emulsion, foam, cream or gel.

## Revendications

**1.** Utilisation d'un composé correspondant à la formule (I) et/ou à la formule (II)

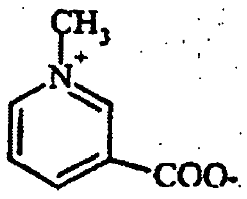

(I)

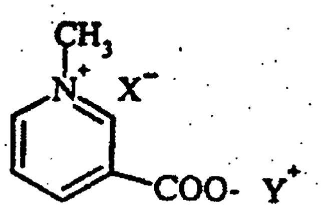

(II)

y compris de ses sels, formules dans lesquelles $X^-$ et $Y^+$ représentent un anion ou, respectivement, un cation organique ou inorganique quelconque, mono- ou plurivalent, physiologiquement sans inconvénient, dans une composition destinée à la réparation, au renforcement, à la restructuration et à la protection contre des endommagements chimiques et mécaniques des cheveux humains.

**2.** Utilisation selon la revendication 1, **caractérisée en ce que** les composés correspondant à la formule (I) et/ou à la formule (II) sont d'origine naturelle ou synthétique.

**3.** Utilisation selon la revendication 1 ou 2, **caractérisée en ce que** les composés correspondant à la formule (I) et/ou à la formule (II) sont présents sous forme de composant d'un extrait naturel de plante.

**4.** Utilisation selon les revendications 1 à 3, **caractérisée en ce que** la substance active en tant que composé de formule (II) est présente sous sa forme de bétaïne pure ou sous forme de sel.

**5.** Utilisation selon les revendications 1 à 4, **caractérisée en ce que** $X^-$ est choisi dans le groupe constitué par le formiate, le tartrate, l'oxalate, l'aspartate, le glutamate, l'acétate, le citrate ou bien des radicaux inorganiques tels que chlorure, bromure, iodure, sulfate, hydrogénosulfate, phosphate, mono- ou dihydrogénophosphate, hydroxyde, carbonate, nitrate et **en ce que** $Y^+$ est choisi dans le groupe constitué par les protons, les métaux alcalins, de préférence le lithium, le sodium, le potassium, les métaux alcalino-terreux, de préférence le calcium et le magnésium, les métaux des groupes B, de préférence l'aluminium, le fer, le zinc, le cuivre, le manganèse, l'argent, des groupes ammonium ou des amines primaires, secondaires, tertiaires ou quaternaires, des hydrazides ou les groupes hydroxylammonium.

**6.** Utilisation selon l'une quelconque des revendications 1 à 5, dans le cas de cheveux fatigués et/ou abîmés.

**7.** Utilisation selon l'une quelconque des revendications 1 à 6, **caractérisée en ce qu'**on met la composition en contact avec les cheveux avant, pendant ou après une exposition des cheveux à des agents nocifs physiques et/ou chimiques.

**8.** Utilisation selon les revendications 1 à 7, dans une composition de prétraitement avant un traitement physique et/ou chimique des cheveux.

**9.** Utilisation selon l'une quelconque des revendications 1 à 8, pour éviter ou réduire des endommagements de la structure interne ou pour réparer (restructurer) les cheveux.

**10.** Utilisation selon l'une quelconque des précédentes revendications 1 à 9, **caractérisée en ce que** le traitement chimique comprend une teinture, un nuançage, une décoloration ou une mise en forme permanente.

**11.** Utilisation selon l'une quelconque des revendications 1 à 10, pour le traitement cosmétique de cheveux sensibles, cassants et/ou fins.

**12.** Utilisation selon l'une quelconque des revendications 1 à 11, **caractérisée en ce que** les composés correspondant

à la formule (I) et/ou à la formule (II) sont contenus dans la composition en une quantité de 0,001 à 30,0 % en poids, par rapport à la quantité totale.

**13.** Utilisation selon l'une quelconque des revendications 1 à 12, **caractérisée en ce que** les composés correspondant aux formules (I) et (II) sont contenus dans la composition en une quantité de 0,05 à 10,0 % en poids, par rapport à la quantité totale.

**14.** Utilisation selon l'une quelconque des revendications 1 à 13, **caractérisée en ce que** la composition se trouve sous forme de solution aqueuse ou aqueuse-alcoolique, d'émulsion, de mousse, de crème ou de gel.

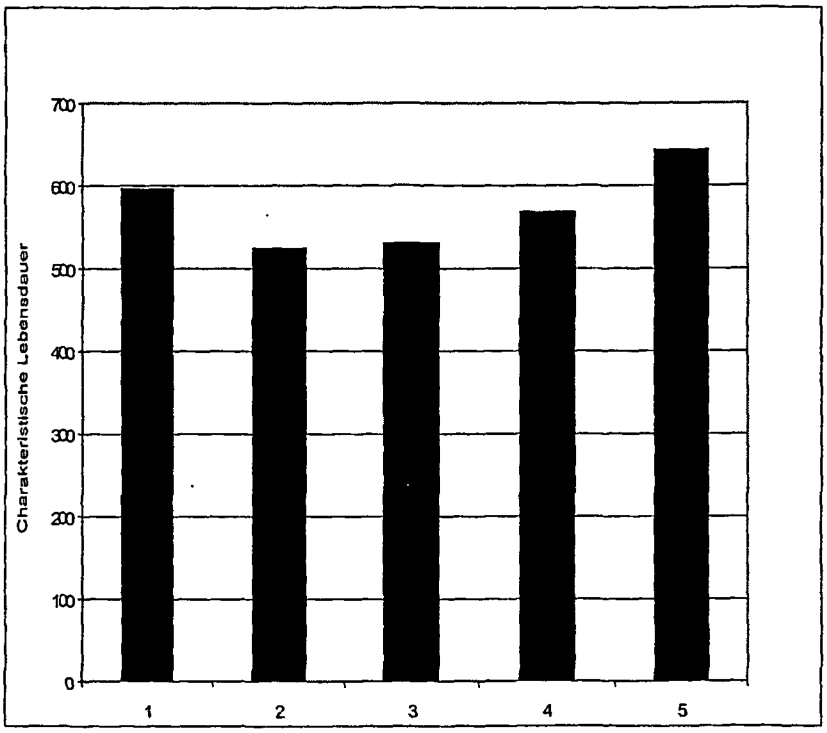
Charakteristische Lebensdauer
700
600
500
400
300
200
100
0
1
2
3
4
5

*Fig.1*

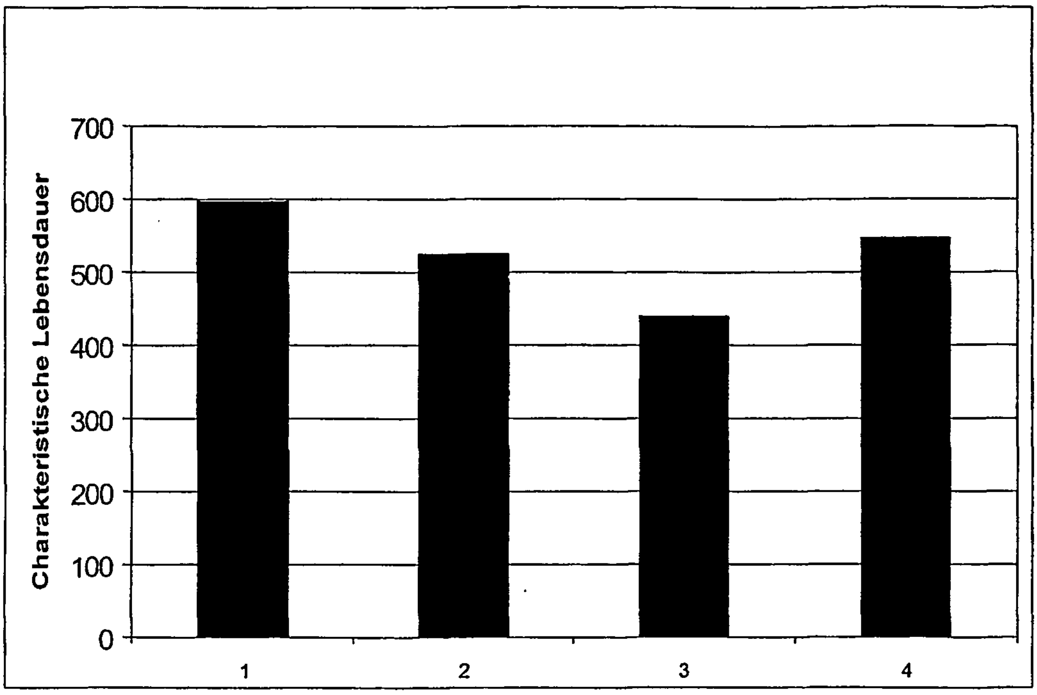
Charakteristische Lebensdauer
700
600
500
400
300
200
100
0
1
2
3
4

*Fig.2*

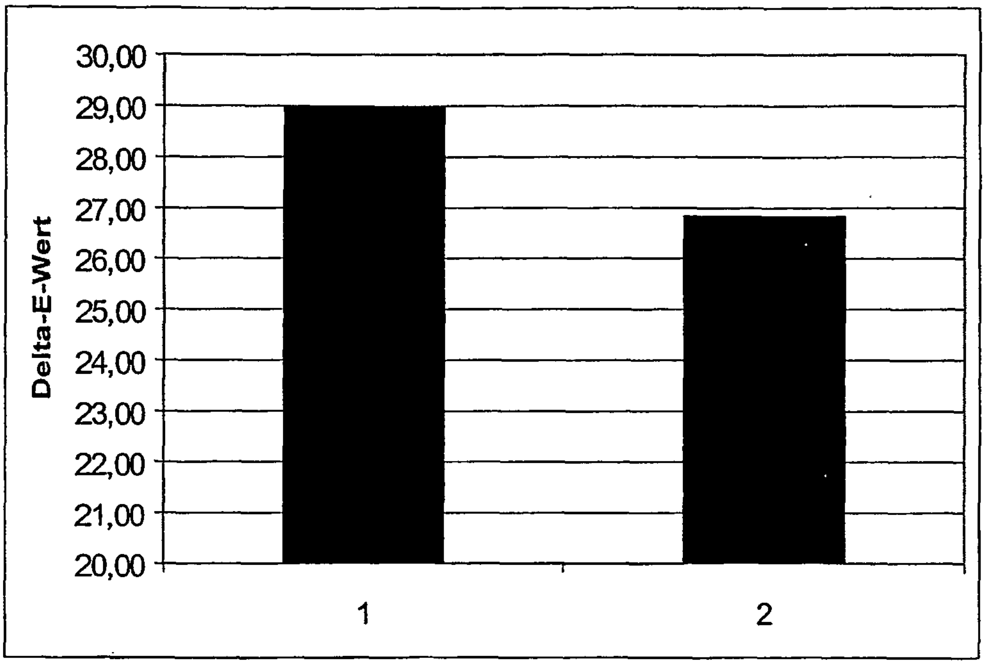
Delta-E-Wert
30,00
29,00
28,00
27,00
26,00
25,00
24,00
23,00
22,00
21,00
20,00
1
2

*Fig.3*

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0040559 A1
- WO 9012560 A1
- WO 9911222 A